# EUROPEAN PATENT SPECIFICATION

(11) **EP 2 299 918 B1**
(45) Date of publication and mention of the grant of the patent: **22.03.2017**
(21) Application number: 09739229.4
(22) Date of filing: 30.04.2009
(51) Int. Cl.: A61B 17/68, A61B 17/80, A61F 2/08

(54) **MOUNTS FOR IMPLANTABLE EXTRA-ARTICULAR SYSTEMS**
HALTERUNGEN FÜR IMPLANTIERBARE EXTRAARTIKULÄRE SYSTEME
SUPPORTS POUR SYSTÈMES EXTRA-ARTICULAIRES IMPLANTABLES

(30) Priority: 30.04.2008 US 49191; 30.04.2008 US 112659
(43) Date of publication of application: 30.03.2011
(73) Proprietor: Moximed, Inc., Hayward, CA 94545 (US)
(72) Inventor: CLIFFORD, Anton, G., Mountain View, CA 94040 (US); LANDRY, Michael, E., Austin, TX 78746 (US); SLONE, Clinton, N., San Francisco, CA 94118 (US); MAKOWER, Joshua, Los Altos, CA 94022 (US)
(74) Representative: V.O.
(86) International application number: PCT/US2009/002697
(87) International publication number: WO 2009/134426

(56) References cited:
- EP-A1- 2 032 056
- WO-A-2005/039454
- WO-A-2008/039777
- DE-A1- 4 018 273
- GB-A- 2 250 919
- US-A- 4 246 660
- US-A1- 2005 080 416
- US-A1- 2008 275 560

## Description

### FIELD OF EMBODIMENTS

Various embodiments disclosed herein relate to structure for attachment to body anatomy, and more particularly, towards approaches for providing mounting members for implantable extra-articular systems.

### BACKGROUND

Joint replacement is one of the most common and successful operations in modern orthopaedic surgery. It consists of replacing painful, arthritic, worn or diseased parts of a joint with artificial surfaces shaped in such a way as to allow joint movement. Osteoarthritis is a common diagnosis leading to joint replacement. Such procedures are a last resort treatment as they are highly invasive and require substantial periods of recovery. Total joint replacement, also known as total joint arthroplasty, is a procedure in which all articular surfaces at a joint are replaced. This contrasts with hemiarthroplasty (half arthroplasty) in which only one bone's articular surface at a joint is replaced and unincompartmental arthroplasty in which the articular surfaces of only one of multiple compartments at a joint (such as the surfaces of the thigh and shin bones on just the inner side or just the outer side at the knee) are replaced. Arthroplasty as a general term, is an orthopaedic procedure which surgically alters the natural joint in some way. This includes procedures in which the arthritic or dysfunctional joint surface is replaced with something else, procedures which are undertaken to reshape or realigning the joint by osteotomy or some other procedure. As with joint replacement, these other arthroplasty procedures are also characterized by relatively long recovery times and their highly invasive procedures. A previously popular form of arthroplasty was interpositional arthroplasty in which the joint was surgically altered by insertion of some other tissue like skin, muscle or tendon within the articular space to keep inflammatory surfaces apart. Another previously done arthroplasty was excisional arthroplasty in which articular surfaces were removed leaving scar tissue to fill in the gap. Among other types of arthroplasty are resection(al) arthroplasty, resurfacing arthroplasty, mold arthroplasty, cup arthroplasty, silicone replacement arthroplasty, and osteotomy to affect joint alignment or restore or modify joint congruity. When it is successful, arthroplasty results in new joint surfaces which serve the same function in the joint as did the surfaces that were removed. Any chodrocytes (cells that control the creation and maintenance of articular joint surfaces), however, are either removed as part of the arthroplasty, or left to contend with the resulting joint anatomy. Because of this, none of these currently available therapies are chondro-protective.

A widely-applied type of osteotomy is one in which bones are surgically cut to improve alignment. A misalignment due to injury or disease in a joint relative to the direction of load can result in an imbalance of forces and pain in the affected joint. The goal of osteotomy is to surgically re-align the bones at a joint and thereby relieve pain by equalizing forces across the joint. This can also increase the lifespan of the joint. When addressing osteoarthritis in the knee joint, this procedure involves surgical re-alignment of the joint by cutting and reattaching part of one of the bones at the knee to change the joint alignment, and this procedure is often used in younger, more active or heavier patients. Most often, high tibial osteotomy (HTO) (the surgical re-alignment of the upper end of the shin bone (tibia) to address knee malalignment) is the osteotomy procedure done to address osteoarthritis and it often results in a decrease in pain and improved function. However, HTO does not address ligamentous instability - only mechanical alignment. HTO is associated with good early results, but results deteriorate over time.

Other approaches to treating osteoarthritis involve an analysis of loads which exist at a joint. Both cartilage and bone are living tissues that respond and adapt to the loads they experience. Within a nominal range of loading, bone and cartilage remain healthy and viable. If the load falls below the nominal range for extended periods of time, bone and cartilage can become softer and weaker (atrophy). If the load rises above the nominal level for extended periods of time, bone can become stiffer and stronger (hypertrophy). Finally, if the load rises too high, then abrupt failure of bone, cartilage and other tissues can result. Accordingly, it has been concluded that the treatment of osteoarthritis and other bone and cartilage conditions is severely hampered when a surgeon is not able to precisely control and prescribe the levels of joint load. Furthermore, bone healing research has shown that some mechanical stimulation can enhance the healing response and it is likely that the optimum regime for a cartilage/bone graft or construct will involve different levels of load over time, e.g. during a particular treatment schedule. Thus, there is a need for devices which facilitate the control of load on a joint undergoing treatment or therapy, to thereby enable use of the joint within a healthy loading zone.

Certain other approaches to treating osteoarthritis contemplate external devices such as braces or fixators which attempt to control the motion of the bones at a joint or apply cross-loads at a joint to shift load from one side of the joint to the other. A number of these approaches have had some success in alleviating pain but have ultimately been unsuccessful due to lack of patient compliance or the inability of the devices to facilitate and support the natural motion and function of the diseased joint. The loads acting at any given joint and the motions of the bones at that joint are unique to the body that the joint is a part of. For this reason, any proposed treatment based on those loads and motions must account for this variability to be universally successful. The mechanical approaches to treating osteoarthritis have not taken this into account and have consequently had limited success.

Prior approaches to treating osteoarthritis have also failed to account for all of the basic functions of the various structures of a joint in combination with its unique movement. In addition to addressing the loads and motions at a joint, an ultimately successful approach must also acknowledge the dampening and energy absorption functions of the anatomy, and be implantable via a minimally invasive technique. Prior devices designed to reduce the load transferred by the natural joint typically incorporate relatively rigid constructs that are incompressible. Mechanical energy (E) is the action of a force (F) through a distance (s) (i.e., E=F^{x}s). Device constructs which are relatively rigid do not allow substantial energy storage as the forces acting on them do not produce substantial deformations - do not act through substantial distances - within them. For these relatively rigid constructs, energy is transferred rather than stored or absorbed relative to a joint. By contrast, the natural joint is a construct comprised of elements of different compliance characteristics such as bone, cartilage, synovial fluid, muscles, tendons, ligaments, etc. as described above. These dynamic elements include relatively compliant ones (ligaments, tendons, fluid, cartilage) which allow for substantial energy absorption and storage, and relatively stiffer ones (bone) that allow for efficient energy transfer.. The cartilage in a joint compresses under applied force and the resultant force displacement product represents the energy absorbed by cartilage. The fluid content of cartilage also acts to stiffen its response to load applied quickly and dampen its response to loads applied slowly. In this way, cartilage acts to absorb and store, as well as to dissipate energy.

With the foregoing applications in mind, it has been found to be necessary to develop effective structures for mounting to body anatomy. Such structures should conform to body anatomy and cooperate with body anatomy to achieve desired load reduction, energy storage, and energy transfer. These structures should include mounting means for attachment of complementary structures across articulating joints.

For these implant structures to function optimally, they must not cause a disturbance to apposing tissue in the body, nor should their function be affected by anatomical tissue and structures impinging on them. Moreover, there is a need to reliably and durably connect a link or an energy absorbing structure at an interventional site and to provide a durable surface for articulating motion without restricting natural ranges of motion of body anatomy. Therefore, what is needed is an approach which addresses both joint movement and varying loads as well as complements underlying anatomy and provides an effective mount for a link or energy manipulating assembly.
Publication US 4246660 discloses a prosthetic ligament device comprising an elastic element securable to the underlying bone structure by means of a quick release bayonet-type fitting allowing rotational movement.
Publication WO 2008/039777 discloses a trans-connector for joining adjacent longitudinal spinal rods. The trans-connector includes a pain of bone fixation coupling elements with a bridge member in between. The bridge member can be adjustable.

### SUMMARY

Briefly, and in general terms, the present disclosure is directed to mounting components that are used in connection with implantable extra-articular systems. The mounting components are intended to provide reliable and durable connections. The components are also intended to provide a durable bearing surface and a secure engagement between moving parts without substantially restricting ranges of motion as well as be removable and/or adjustable.

According to one embodiment, the mount includes a body having a first portion opposite a second portion, the second portion providing an articulating connection. The mount can further include a coupling connection in the form of a locking stem that is configured at the first portion of the mount for attaching the mount to a base component. The mount can also include a multi-dimensional articulating connection component (for example, a socket for receiving a ball) defining a bearing surface formed at the second portion of the mount. The body of the mount offsets the multi-dimensional articulating connection component away from body anatomy when coupled to the base component. The mount also orients and aligns a link or absorber that is coupled to the articulating connection component. In one aspect, the link or absorber to bearing surface connection is arranged so that the link or absorber is easily inserted or removed. In another aspect, the contemplated structure is intended to withstand maximum stresses at maximum loading conditions for a high number of cycles.

In another embodiment, the mount includes a locking coupling connection that is positioned at a first end of the mount for attaching the mount to a base component. The mount also includes an adjustable multi-dimensional articulating connection component slidably affixed to a second end of the mount, the articulating connection defining a bearing surface. The body of the mount offsets the multi-dimensional articulatable connection component away from body anatomy when coupled to the base component. The mount also orients and aligns a link or absorber that is coupled to the articulatable connection component.

The only embodiments falling within the scope of the claims include deformable material which facilitates an engagement between mounting structure and a base component. Such deformable material is configured as a separate sleeve.
Additionally, the sleeve can include surface ridges facilitating relative movement between parts as well as a desired locking engagement.

In one particular embodiment, a mount component is formed from cobalt chromium. Additionally, a sleeve can be made from titanium and portions of the mount can be coated with a ceramic material. Tapered structure is employed to act as a funnel to aid in guiding parts to proper positions. In this regard, one or more of a locking stem, deformable sleeve or stem receiving hole can be tapered. Tabs and receiving slots are also contemplated to aid in proper orientation between parts.

Moreover, a highly refined, polished surface finish is contemplated for the bearing surfaces. Tight tolerances respecting diameter clearances with structure received by the bearing surface as well as the spherecity of the pocket defined by the bearing surfaces are required in certain applications. Symmetrical as well as asymmetrical bearing surfaces are also contemplated for example in a knee application the surfaces accomplish articulating limb flexion/extension rotation of up to 130°, varus/valgus rotation up to 10° and internal/external rotation of 75°.

Other features and advantages will become apparent from the following detailed description, taken in conjunction with the accompanying drawings, which illustrate by way of example, the features of the various embodiments.

### BRIEF DESCRIPTION OF THE DRAWINGS

FIG. 1 is a perspective view, depicting a preferred embodiment of an implantable mechanical energy absorber system with a protective housing or sheath removed to show the components;
FIG. 2 is an enlarged perspective view, depicting a first mount component;
FIG. 3 is a perspective view, depicting the first mount component rotated with respect to FIG. 2;
FIG. 4 is a perspective view, depicting a socket portion of the first mount component of FIG. 2;
FIG. 5 is an enlarged perspective view, depicting a second mount component;
FIG. 6 is a perspective view, depicting the second mount component rotated with respect to FIG. 5;
FIG. 7 is a perspective view, depicting a socket portion of the second mount component;
FIG. 8 is a perspective view, depicting the system of FIG. 1 with an absorber element and first and second mount components removed;
FIG. 9 is an enlarged side view, depicting the energy absorber element removed from FIG. 8;
FIG. 10 is an enlarged perspective view, depicting a deformable sleeve;
FIG. 11 is a perspective view of one embodiment of a mounting member coupled to a base component that is fixed to a bone;
FIG. 12 is a back perspective view of one embodiment of an extra-articular implantable mechanical energy absorbing system coupled to base components via mounting members;
FIG. 13A is a top perspective view of one embodiment of a coupling connection between a mounting member and a base component;
FIG. 13B is a cross-section view of the coupling connection of FIG. 13A;
FIG. 13C is a top view of another embodiment of a lockable connection between a mounting member and a base component in an unlocked position;
FIG. 13D is a top view of the lockable connection of FIG. 13C in a locked position;
FIG. 14A is a perspective view of another embodiment of a coupling connection between a base component and a mounting member;
FIG. 14B is a perspective view of yet another embodiment of a coupling connection between a base component and a mounting member;
FIG. 15 is a perspective view of another embodiment of a coupling connection between a base component and a mounting member;
FIG. 16 is a perspective view of another embodiment of a coupling connection between a base component and a mounting member;
FIG. 17A is an exploded view of another embodiment of a coupling connection between a base component and a mounting member;
FIG. 17B is a top view of the coupling connection of FIG. 17A fully assembled;
FIG. 18A is a top view of one embodiment of a articulatingable connection usable with a mounting member;
FIG. 18B is a top view of another embodiment of a articulatingable connection usable with a mounting member;
FIG 18C is a perspective view of yet another embodiment of a articulatingable connection usable with a mounting member;
FIG. 19 is a perspective view of another embodiment of a articulatingable connection provided on a mounting member;
FIG. 20 is a side view of one embodiment of an adjustable articulating connection provided on a mounting member;
FIG. 21 is a side view of another embodiment of an adjustable articulating connection provided on a mounting member;
FIG. 22A is a cross-sectional view of yet another embodiment of an adjustable articulating connection provided on a mounting member;
FIG. 22B is a perspective view of one embodiment of a shim used in the adjustable articulating connection of FIG. 22A;
FIG. 23 is a cross-sectional view of another embodiment of an adjustable articulating connection provided on a mounting member;
FIG. 24 is a cross-sectional view of another embodiment of an adjustable articulating connection provided on a mounting member;
FIG. 25A is a cross-sectional view of another embodiment of an adjustable articulating connection provided on a mounting member;
FIG. 25B is a top view of a lever used with the adjustable articulating connection of FIG. 25A;
FIG. 25C is a side view of a ball component used with the adjustable articulating connection of FIG. 25A;
FIG. 26A is a cross-sectional view of another embodiment of an adjustable articulating connection provided on a mounting member;
FIG. 26B is a cross-sectional view of a locking washer of FIG. 26A in a locked position;
FIG. 26C is a cross-sectional view of a locking washer of FIG. 26A in an unlocked position;
FIG. 27A is a side view of another embodiment of an adjustable articulating connection provided on a mounting member;
FIG. 27B is a top perspective view of the adjustable articulating connection provided on a mounting member of FIG. 27A;

### DETAILED DESCRIPTION

Various disclosed embodiments are directed to mounting members for implantable medical devices. In one particular aspect, the mounting members are attachable to base components that are mounted to a bone as well as to a link mechanism for an implantable extra-articular system. The mounting member includes a first portion performing a coupling connection for mounting to the base component. A second portion of the mounting member includes a socket for coupling to the link mechanism. According to one approach, the socket is a fixed mechanism. In other embodiments, a portion of the socket mechanism is one or more of adjustable and removable. The two part base/mounting member components provide a method for good attachment of the base to the bone and a more simple surgical technique for installing the link. It also allows a protective sheath (not shown) and/or the wear components of the link/mount assembly to be removeable and/or replaceable without removing or replacing the base components. It further allows the wear components of the link/mount assembly and the base components to be different materials. For example, the base components can be titanium or titanium alloy which promote osteo-integration and the wear components can be much harder materials such as cobalt chrome (e.g., Biodur CCM Plus), ceramic, or other durable materials that produce a minimal amount of particulate material or, if particulate material is generated, the smallest size of particulate material.

Thus, the disclosed mounting assemblies provide reliable and durable connections between moving parts. Since moving parts wear, the contemplated approaches facilitate easy and reliable removal and replacement of wearing elements. Accordingly, removal methods, features and tools are contemplated. Moreover, there has been a recognition of varying kinematics of an implant by altering juxtapositional relationships with anatomy and the same has been considered in certain of the mount approaches.

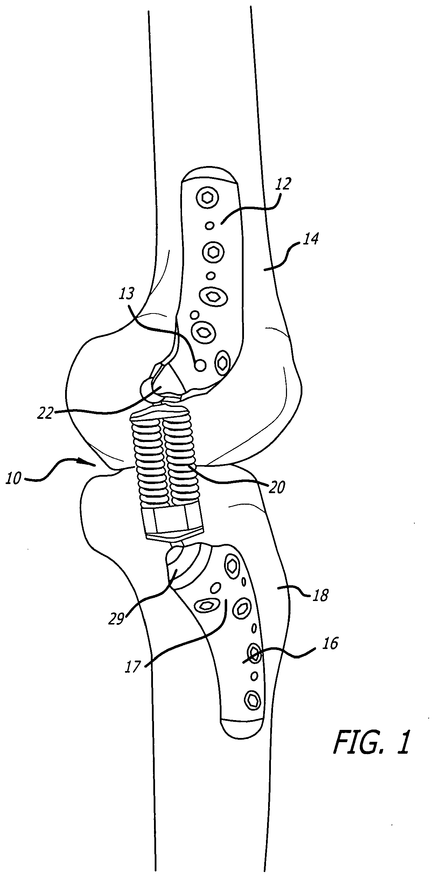

Referring now to the drawings, wherein like reference numerals denote like or corresponding parts throughout the drawings and, more particularly to FIGS. 1-27B, there are shown various embodiments of a mounting member. With specific reference to FIGS. 1-10, there is shown a preferred embodiment of an implantable mechanical energy absorbing system 10. In the application shown, the system is extra-articular in nature in that it is connected exterior of the joint capsule and across a knee joint; however, features of the present disclosure can be applied to various body anatomy.

In the particular embodiment depicted, the implantable mechanical energy absorbing system 10 includes a femoral base component 12 attached to a femur 14 and a tibial base component 16 attached to a tibia 18. Configured between the bases 12, 16 is an absorber 20. To connect the absorber 20 to the bases 12, 16, in one approach, a first mount 22 is employed to attach a first end of the absorber 20 to the femoral base 12 and a second mount 24 is utilized to attach a second end of the absorber to the tibial base 16.

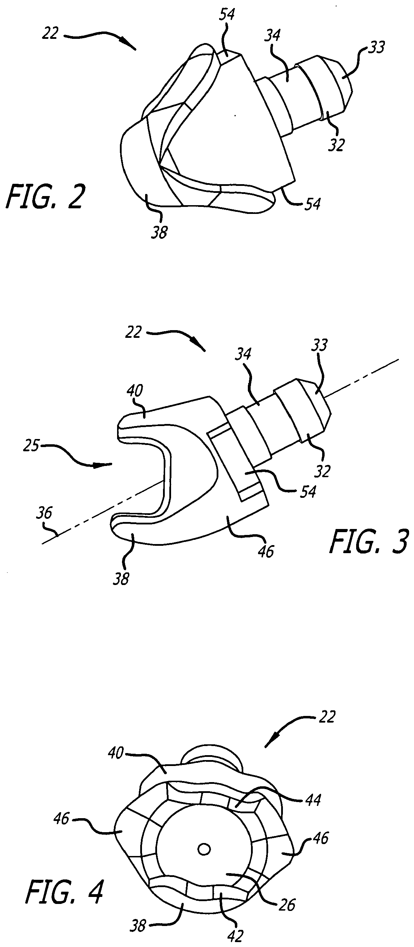

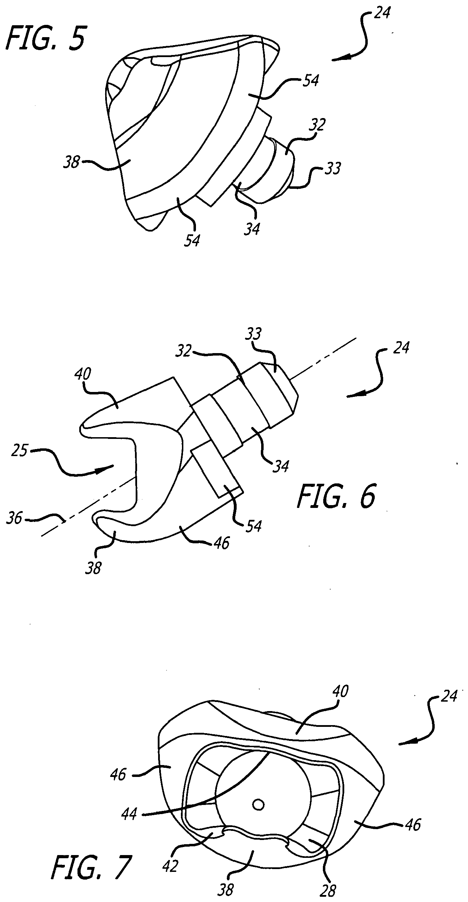

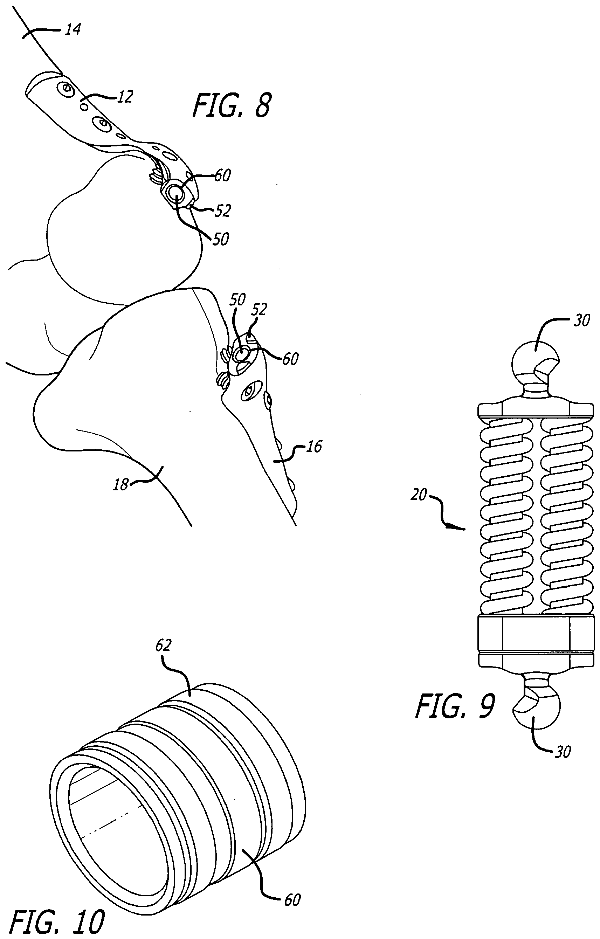

The mounts 22, 24 are designed to reliably and durably connect the absorber 20 to the base components 12, 16. Additionally, the mounts 22, 24 define a socket 25 configured to provide durable bearing surfaces 26, 28 (best seen in FIGS. 4 and 7) for a ball joint 30 (See FIG. 9) configured at the ends of the absorber 20. Moreover, the structure of the mounts 22, 24 provide a secure engagement of the ball joints 30 within the sockets 25 without overly restricting ranges of motion.

Each of the mounts 22, 24 define slightly different structures but have aspects in common. The mounts 22, 24 each include a generally cylindrical locking stem 32 having a tapered terminal end 33. Moreover, the stems can include a mid-section 34 having a slightly reduced diameter. Also, in one approach, the overall profile of the stems can be tapered along its length toward their terminal ends.

The stems 32 extend from a base of the socket portion 25 of the mounts 22, 24. A longitudinal axis 36 extends through the stem 32. Extending in an opposite direction from the stem are curved walls which define the socket portion 25. Although the walls are contiguous with each other, for purposes of description, two walls 38, 40 are identified. Moreover, while the walls of the first and second mounts 22, 24 have different configurations, they are described together here to the extent they have common features. It is to be recognized that the walls 38, 40 are sized and shaped for receiving oppositely oriented ball joints 30 extending from the absorber 20. In this regard, the hooked shape of the ball joints 30 facilitate the insertion of the ball joint 30 within the socket 25. Moreover, the inter-relationship of the shapes of the ball joints 30 and the socket 25 function to securely retain the structures together when a complete implantation energy absorber 10 is attached at an interventional site.

The first and second curved walls 38, 40 include interior surfaces which, in conjunction, define the bearing surface 26 of the socket 25. As such, the walls 38, 40 provide the bearing surface 26 with a contour defining a portion of a spherical surface. In one aspect, this spherical surface is machined to provide a 0.0508 mm (0.002 inch) diametrical clearance with the ball joint 30. A highly fine 2 surface finish is contemplated for the bearing surface.

The curved walls 38, 40 also define a gap or opening to the socket 25. The opening is off-center when considering the longitudinal axis 36 extending through the mount. To accomplish this, the first wall 38 has a longer longitudinal, curved dimension as compared to the second wall 40. As such, the walls define an asymmetric socket. In an alternative embodiment, the walls are mirrored images and define a symmetrical socket. Moreover, each of the walls 38, 40 include curved reliefs 42, 44 removed from the respective walls, a first relief 42 formed in the first wall 38 being deeper than a second relief 44 of the second wall 40. A transition structure 46 having an irregular surface area (best seen in FIGS. 3 and 6) is formed between the walls 38, 40. Since the base of the tibial mount 24 is larger than the base of the femoral mount 22, the transition structure 46 of the tibial mount 24 assumes a larger area than that of the femoral mount

It is the specific shapes of this transitional structure area 46 as well as that of the curved walls 38, 40 which define the opening to the socket 25. In this regard, the curved walls 38, 40 and the transitional area 46 provide the mounts 22, 24 with structure to secure the ball joints without overly restraining ranges of motion. In one particular aspect, the mounts 22, 24 of system 10 affixed to a knee joint can provide 130 degrees of flexion/extension rotation, 10 degrees of varus/valgus rotation and an internal/external rotation of 75 degrees. The mount connector is also desired to withstand a greater than 118 kp (260 pound) pullout force and contact stress exceeding 325 MPa.

Additionally, the walls 38, 40 are configured to permit easy insertion and removal of the ball joints upon twisting of the same relative to the sockets 25 during assembly or disassembly. The mounts 22, 24 can be formed from any durable material. In a preferred approach, the mounts are machined from CoCr Biodur CCM plus material. In certain approaches, a ceramic material can be coated or otherwise formed on exterior surfaces of the mounts 22, 24 such as within the socket 25. Ceramic materials may be used to minimize the generation of particulate matters due to prolonged interfacing between parts. Moreover, in certain applications, the structures are contemplated to be designed to maintain functionality for greater than two million loading cycles.

In order to accomplish a secure attachment between the mounts 22, 24 and the bases 12, 16, an interference fit can be employed. As stated, the stem 32 of the mounts 22, 24 can be tapered. Also, recesses 50 formed in the bases 12, 16 can be tapered to a different degree. Moreover, the stems 32 and recesses 30 can assume other interfering structures such one being tapered with respect to the other, the first structure having a straight profile. In either approach, it is contemplated that a connection between the pieces be facilitated by a funnelling action. Also, tabs 52 can be formed on the bases which register into recesses provided in the mounts to aid in proper orientation between the parts. It is also to be noted that such structures can be placed on opposite parts or each part can include tabs and recesses.

In the only embodiment falling within the scope of the claims, a deformable sleeve 60 is included to facilitate accomplishing a secure engagement between the mounts 22, 24 and the bases 12, 16. Thus, the sleeve 60 can act as a sacrificial structure, giving up its original shape to accomplish a locking function. In one embodiment, the sleeve is formed of titanium or a titanium alloy. As with the recesses 50 formed in the bases 12, 16 and the stems 32 themselves, the sleeve 60 can have a tapered profile. The tapered profile can either be on an exterior of the sleeve or within its bore. As shown in FIG. 8, the sleeve can be sized to be placed within the base recesses 50, its internal bore configured to securely receive a stem 30.

In its assembled configuration, the sleeve 30 deforms about the stem 30 and within the base recesses 50. To facilitate its insertion into the recesses 50, the sleeve 30 can include variously spaced and sized annular recesses 62 which present a smaller surface area while the sleeve is moved relative to the recess 50. The slightly reduced mid-section 34 of the stem 30 also aids in both the relative movement and secure engagement between the sleeve 60 and stem

In certain approaches, locking forces ranging between 27.2-204.1 kp (60-450 pounds) or within a smaller range of 27.2-68 kp (60-150 pounds) have been found useful. Since taper lock forces are proportional with taper dimensions and surface area, reducing surface area can result in reducing locking forces. Unlocking forces can be 50-150% of locking forces and controlled in a similar fashion. The mount 22 can be unlocked and removed from base 12 by inserting a pry tool between the joining interface of mount 22 to base 12 or by inserting a tool into the opening 13 on base 12 that allows access to the end 33 on the mount 22 such that rotation of the tool (such as an oval-shaped shaft) acts as a cam to push on the end 33 and force the stem 30 out of the base 12. Similarly, mount 24 can be unlocked and removed from base 16 by inserting a pry tool between the joining interface or by inserting a tool into the opening 17.

Next is described various other embodiments and approaches to mounts. The above described features as well as materials, surface finishes, coatings and design requirements can be incorporated as needed into such approaches.

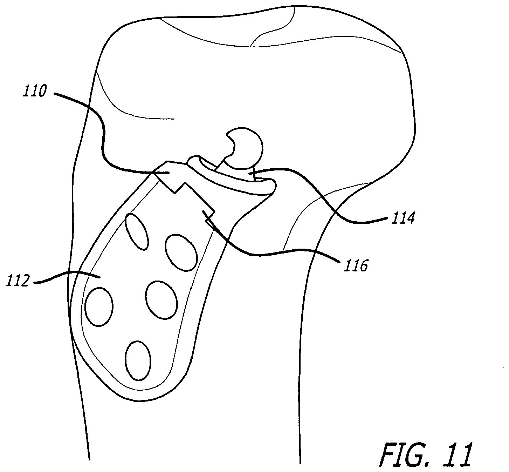

With reference now to FIG. 11, in a further embodiment, a mounting member 110 is coupled to a base component 112 at a first end portion and a link member (not shown) at a second end portion. At the first end, the mounting member 110 includes a coupling connection 116 for securing the mounting member to the base component 112. As shown in FIG. 11, the coupling connection 116 is a dovetail connection. At the second end, the mounting member 110 terminates at a ball component 114, which is one portion of a ball-and-socket mechanism. Alternatively, the mounting member 110 terminates at a socket component at the second end.

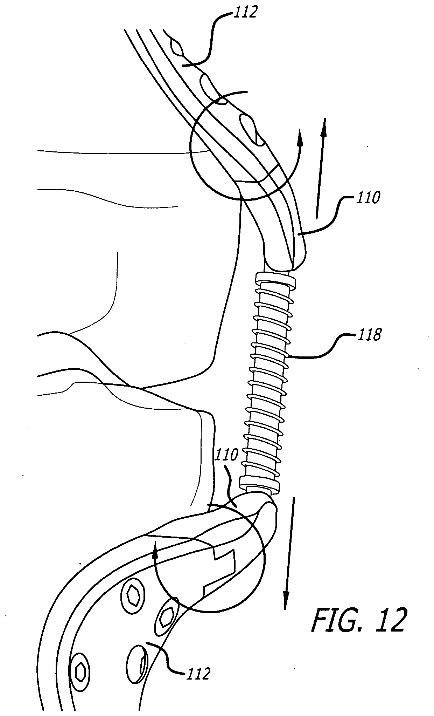

The mounting member 110 as shown in FIG. 11 has a generally tapered shape. The body of the mounting member 110 narrows when moving distally (i.e., the mounting member is wider at the connection point between the base component as compared to the width of the mounting member at the ball component 114). As shown in FIG. 12, the mounting member 110 may also be configured to further offset the ball component 114 (or socket component) away from the bone thereby allowing the link or absorber 118 to avoid bone structures, ligaments, muscles, and the like. The mounting member 110 also allows for proper alignment and orientation of a link or absorber 118 so that the link or absorber can freely move and reduce or remove forces on articulating surfaces of a joint. Comparing the mounting members 110 of FIGS. 11 and 12, the mounting members have different shapes. As those skilled in the art will appreciate, different shapes of the mounting members 110 accommodate different types of links or absorbers and allow varying load reduction at a joint. Accordingly, the link or absorber 118 and the mounting member 110 may be varied to better fit patient needs.

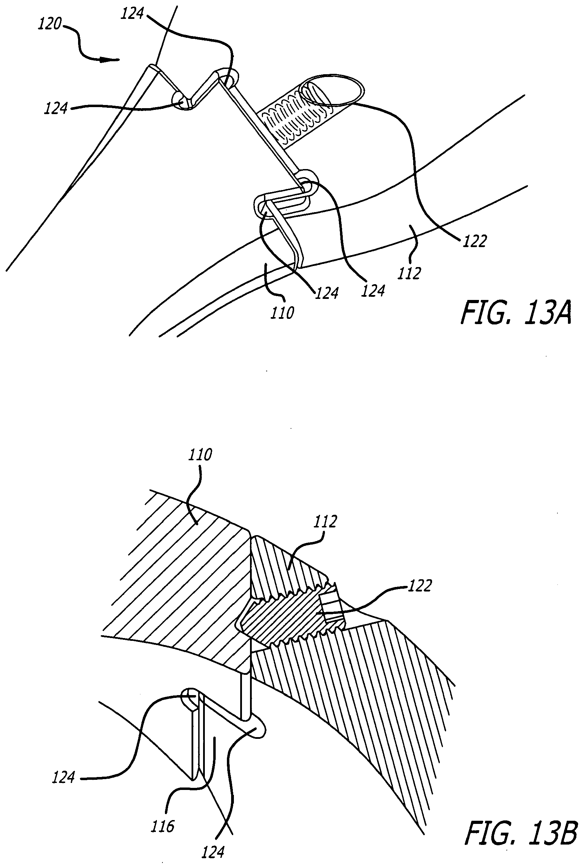

FIGS. 13A-13D illustrate various embodiments of a coupling connection 120 between the mounting member 110 and a base component 112. FIG. 13A illustrates one embodiment of a tapered dovetail connection 120 between the mounting member 110 and a base component 112. As shown in FIG. 13A, the dovetail is provided on the mounting member 110 and a corresponding recess is provided on the base component 112. Alternatively, the dovetail may be provided on the base component 112 and the corresponding recess is provided on the mounting member 110. As shown in FIG. 13A, a set screw 122 is also provided to further secure the dovetail connection 120. Additionally, a plurality of relief cuts 124 are provided on the dovetail connection 120 in order to reduce the stress concentrations at the root of the dovetail. The dovetail can also include rounded edges to further reduce stress concentrations.

FIG. 13B is a cross-sectional view of the dovetail connection 120 of FIG. 13A. As shown in FIG. 13B, the set screw 122 forces the mounting member 110 down onto the base component 112, thereby tightening the dovetail connection 120. The set screw 122 is inserted into the base component 112 at an angle to improve access to tighten or loosen the screw.

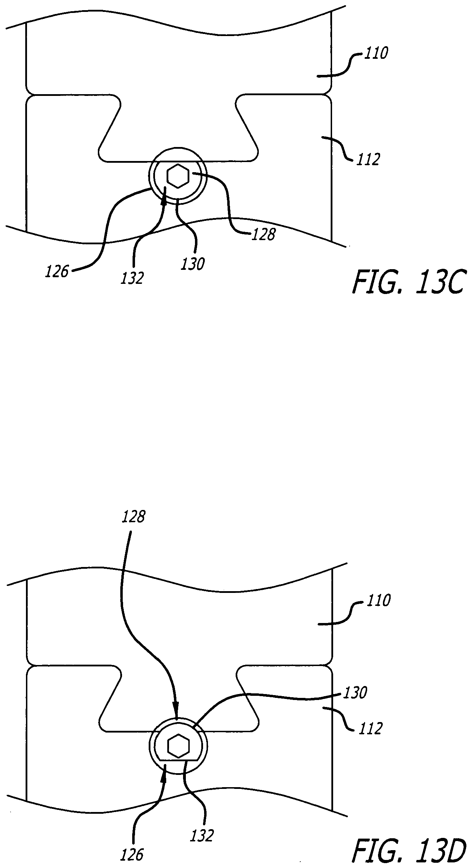

FIG. 13C illustrates another locking dovetail connection 120. The dovetail and corresponding recess are similar as to the dovetail connection shown in FIG. 13A. As shown, the connection 120 includes a recess 126 adjacent to the connection. A captured cam 128 is provided within the recess 126. The cam 128 has a rounded edge 130 and a flattened edge 132. As shown in FIG. 13C, the mounting member 10 may be separated from the base component 112 since the flattened edge 132 of the cam 128 is aligned with the edge of the mounting member 110. As shown in FIG. 13D, the cam 128 can be rotated approximately 90° to 180° to have the rounded edge 130 engage a portion of the mounting member 110 thereby securing the connection between the mounting member 110 and the base component 112.

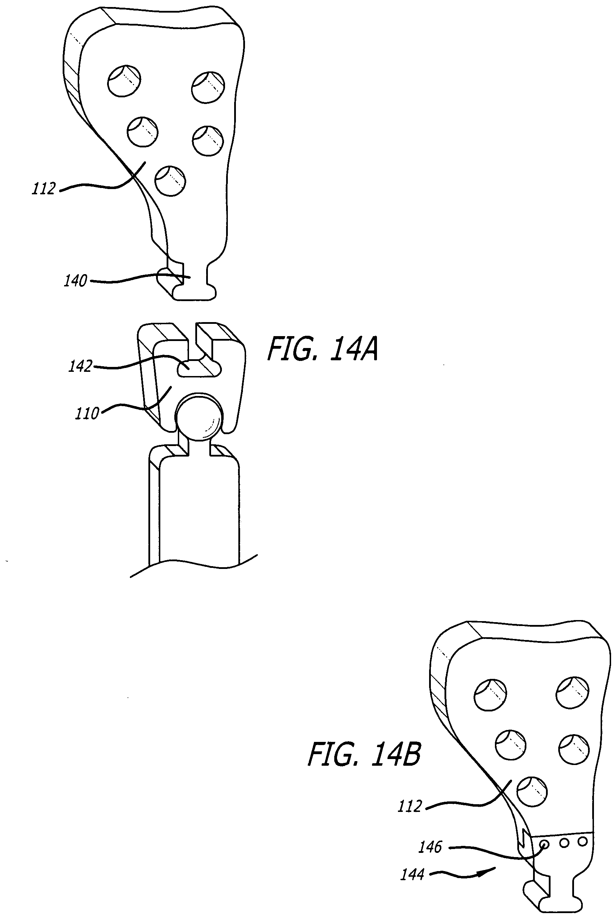

FIG. 14A illustrates another embodiment of a coupling connection 140 between a base component 112 and a mounting member 110. The coupling connection 140 is a snap fit connection between the base component 112 and the mounting member 110. The base component 112 has a shaped protuberance 140 that mates with a corresponding slot 142 on the mounting member 110. In an alternate embodiment, the mounting member 110 includes the shaped protuberance and the corresponding slot is provided on the base component 112. Optionally, the snap connection 40 may incorporate locking mechanisms as shown in FIGS. 13A-13D.

With reference to FIG. 14B, yet another embodiment of a coupling connection 144 between a base component 112 and a mounting member 110 is depicted. As shown, the edges of the mounting member 110 and the base component 112 are overlapping. One or more screws 146 to secure the connection between the mounting member 110 and the base component 112. Again, alternatively, the connection 144 may incorporate locking mechanisms as shown in FIGS. 13A-13D.

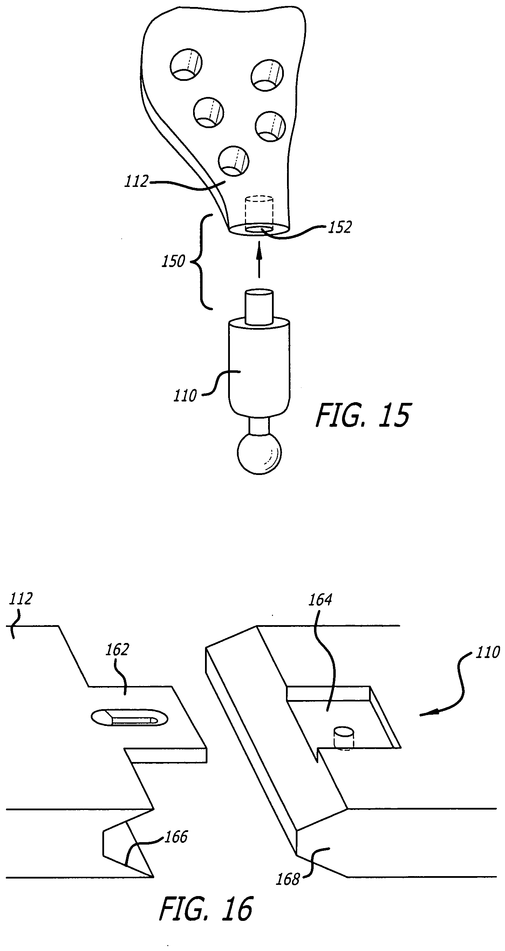

A friction-fit coupling connection 150 approach between a base component 112 and a mounting member 110 is shown in FIG. 15. In this embodiment, the end of the base component 112 includes a bore 152. The mounting member 110 includes a tapered shaft having a first diameter at a first end (at the tip of the shaft) and a second diameter at a second end (at the base of the shaft) where the second diameter is larger than the first diameter. Locking mechanisms can also be incorporated here.

FIG. 16 illustrates another embodiment of a coupling connection 160 between a base component 112 and a mounting member 110. The base component 112 includes a tongue 162 that engages a groove 164 provided on the mounting member 110. Additionally, the base component 112 and the mounting member 110 may include additional interlocking surfaces 166, 168 to further secure the two surfaces together. The tongue 162 includes a tapered locking screw hole 170, and the groove 164 also includes a screw hole. When the tongue 162 and groove 164 are properly aligned, a screw or other fastening means may be used to secure the tongue within the groove.

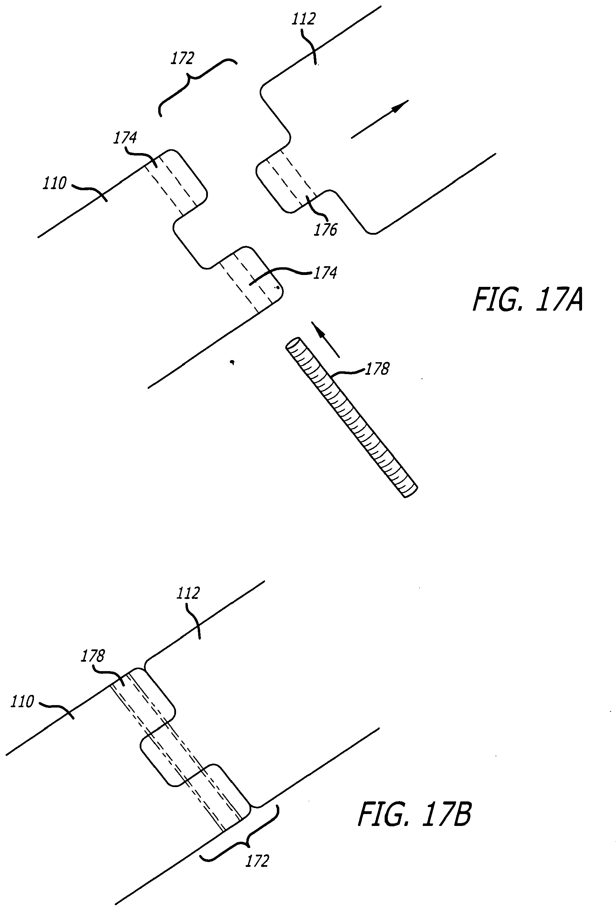

Yet another approach of a coupling connection 172 between a base component 112 and a mounting member 110 is shown in FIGS. 17A-B. Here, the coupling structure 172 is a dovetail connection where the dovetail is provided on the base component 112 and a corresponding recess is provided on the mounting member 110. Alternatively, the dovetail may be provided on the mounting member 110 and the corresponding recess is provided on the base component 112. The dovetail connection includes through holes 174 in the walls of the recess and the dovetail 176. According to one embodiment, a locking pin 178 having locking threads is threaded through the through holes 174, 176 to secure the dovetail connection 172. In another embodiment, the locking pin 178 is tapered, and the locking pin is press fitted through the holes 174, 176 to secure the connection 172.

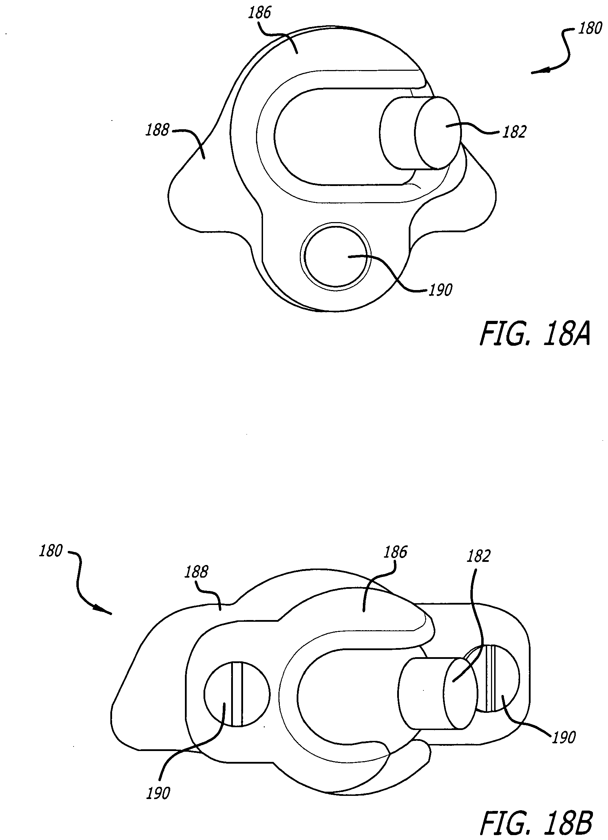

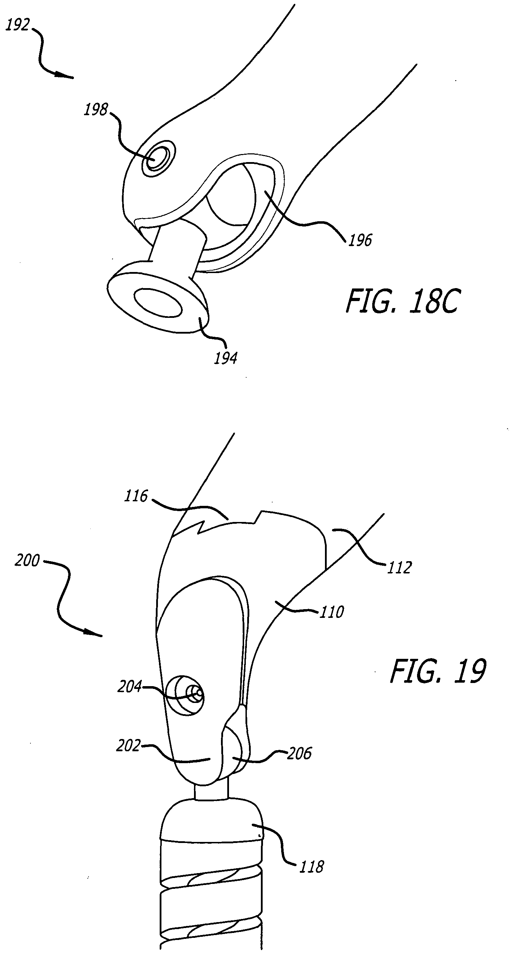

Turning now to FIGS. 18A-18C, various embodiments of a ball-and-socket connection 180 are depicted. As shown, the connection 180 includes a ball component 182 that is secured within a socket that includes a first socket portion 186 that is secured to a second socket component 188 with one or more screws 190. As those skilled in the art will appreciate, any fastening means known or developed in the art may be used to couple to the first and second socket portions 186, 188. FIG. 18C illustrates a universal connection 192 where the ball component 194 is secured within a one-piece socket 196 via a pin 198.

A mounting member 110 terminating at a socket component 200 is shown in FIG. 19. The socket component 200 includes a first portion that is integral with the mounting body and a second portion 202 that may be fastened to the first portion via a screw 204 or other securing means. As shown, a ball component 206 is inserted and secured within the socket component 200. The ball component 206 is coupled to one end of the link or absorber 118.

Referring back to FIG. 11, the ball component 114 is provided on the mounting member 110. In alternate embodiments, the socket portion may be provided on the mounting member as shown in FIG. 12. In other embodiments, a portion of other articulating or articulatinging surfaces as disclosed in U.S. Patent Application No. 11/775,145, filed on July 9, 2007, and published as US2008275558 A1, may be provided on the mounting member 110. Additionally, as shown in FIGS. 11-12, the ball or socket component is fixed to the mounting member 110. Accordingly, the ability to adjust the ball or socket location in these embodiments is dependent upon changing the shape of the mounting member 110 or moving the base component 112.

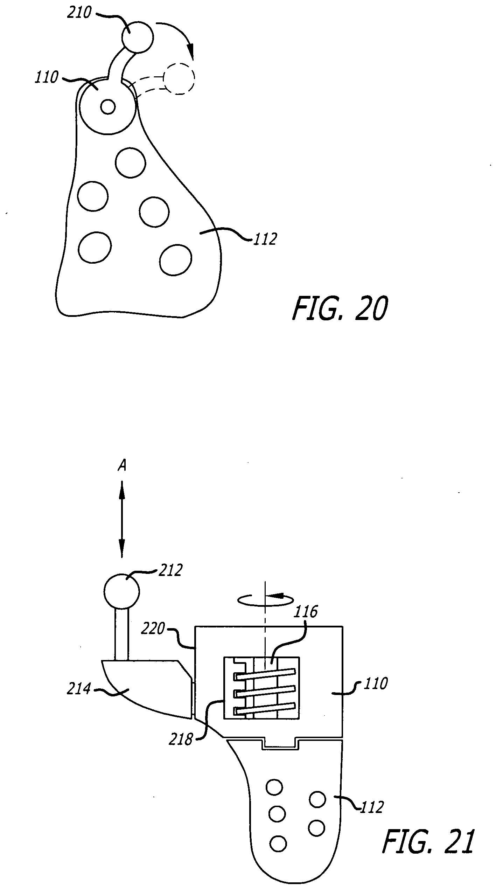

FIGS. 20-27B illustrate various embodiments of a mounting member 110 having an adjustable articulating connection component. The adjustable articulating connection allows a ball or socket component to be moved thereby providing adjustability in the location, alignment, or range of motion of the articulating connection. The adjustability can be used to alter the load carrying capacity of the link or absorber and/or to alter the displacement range of the link or absorber. For example, FIG. 20 illustrates one embodiment of a rotatable mounting member 110 having a ball component 210 at one end of the member. The mounting member 110 is rotatably fixed to the base component 112. Accordingly, rotation of the mounting member 110 allows for shifting the connection point for the ball component 210 and socket component (not shown).

FIG. 21 illustrates another embodiment of a mounting member 110, which is coupled to a base component 112 which has an adjustable articulating connection component. As shown in FIG. 21, the adjustable articulating connection component is a ball component 212, but the ball component may be a socket component in alternate embodiments. The ball component 212 is coupled to a movable arm 214. The movable arm 214 is operably connected to a thumbscrew 216 such that rotation of the thumbscrew relative to the teeth 218 causes the movable arm to translate upwards or downwards as indicated by arrow A. Optionally, detents (not shown) may be provided along the mount surface 220. The detents may be spaced approximately 1 mm apart (or any other fixed distance) thereby providing a plurality of locations for registering the arm 214. As the movable arm 214 passes the detents, an audible click is emitted thereby providing the end user with an indication that the arm (and ball component) is being linearly translated. In alternate embodiments, a rack and pinion mechanism (not shown) may be used to move the ball component 212.

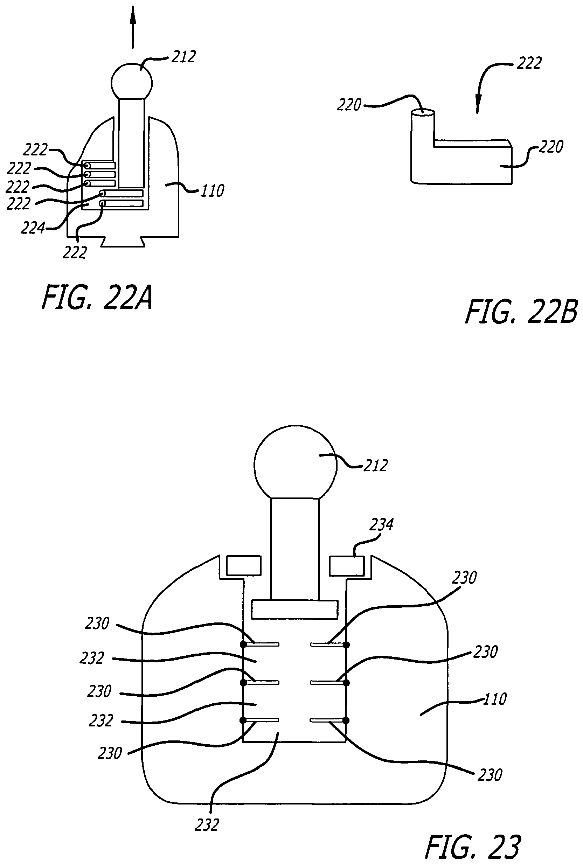

With reference to FIG. 22A, another embodiment of a mounting member 110 having an adjustable articulating connection is depicted. As shown, the mounting member 110 includes a movable ball component 212. In an alternate embodiment, the mounting member 110 includes a socket component in place of the movable ball component 212. A plurality of shims 222 are provided in a recessed area 224 of the mounting member 110. The shims 222 may be slid in the recess to engage or disengage the ball component 212. Accordingly, the ball component 212 is raised to the mounting member 110 as shims 222 are positioned below the ball component. Placement of additional shims 222 engaging the ball component 212 moves the ball component away from the body of the mounting member 110. The shim 222 has a body portion 226 and a lever portion 228. Optionally, a radiopaque coating is applied to the lever portion 228 and/or the body portion 226 so that the shim may be seen under a fluoroscope to visualize the shims after the mounting member 110 has been fixed within a patient.

A mounting member 110 having a movable ball component 212 that is positioned above a plurality of compartments 232 is shown in FIG. 23. In an alternate embodiment, the mounting member 110 can include a socket component in place of the movable ball component 212. The compartments 232 are separated by flexible dividers 230. The dividers 230 are separated at a fixed distance such as, but not limited to, approximately 1 mm. According to one embodiment, one or more of the compartments 232 may be filled with bone paste or other material to set a desired height of the ball component 212 relative to the mounting member 110. Additionally, a retaining member 234 secures the ball component 212 to the mounting member 110.

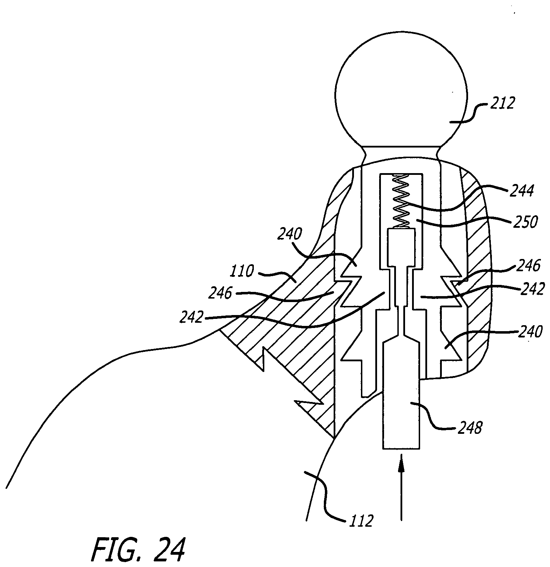

Turning to FIG. 24, a mounting member 110 having a movable ball component 212 is shown. In an alternate embodiment, the mounting member 110 includes a socket component in place of the movable ball component 212. The ball component 212 is coupled to a shaft having a bore 250. The shaft of the ball component 212 is deflectable and may compress and expand when a force is applied to the shaft. The bore 250 also includes an annular stop 242. Additionally, a release pin 248 is slidably coupled within the bore 250. A spring 244 is coupled to the base of the bore 250 at one end and the release pin 248 at a second end. The shaft includes a plurality of rings 242 on the outer diameter. The rings 242 are spaced a fixed distances such as, but not limited to, approximately 1 mm. The ball component 212 is placed within the bore of the mounting member 110. The bore of the mounting member 110 also includes a ring stop 246 that extends into the bore of the mounting member.

In operation, the release pin 248 is depressed until the pin contacts the annular stop 242 with the bore 250. Any additional force causes the ball component 212 to move from one ring 246 to an adjacent ring. As the force is applied to the release pin 248, the walls of the shaft deflect to move around ring stop 246. Alternatively, the release pin 248 may be pulled downward until the stop on the outer diameter of the release pin contacts the annular stop 242. As the downward force is applied to the ball component 212, the ball component may be lowered from one ring 240 to the next ring.

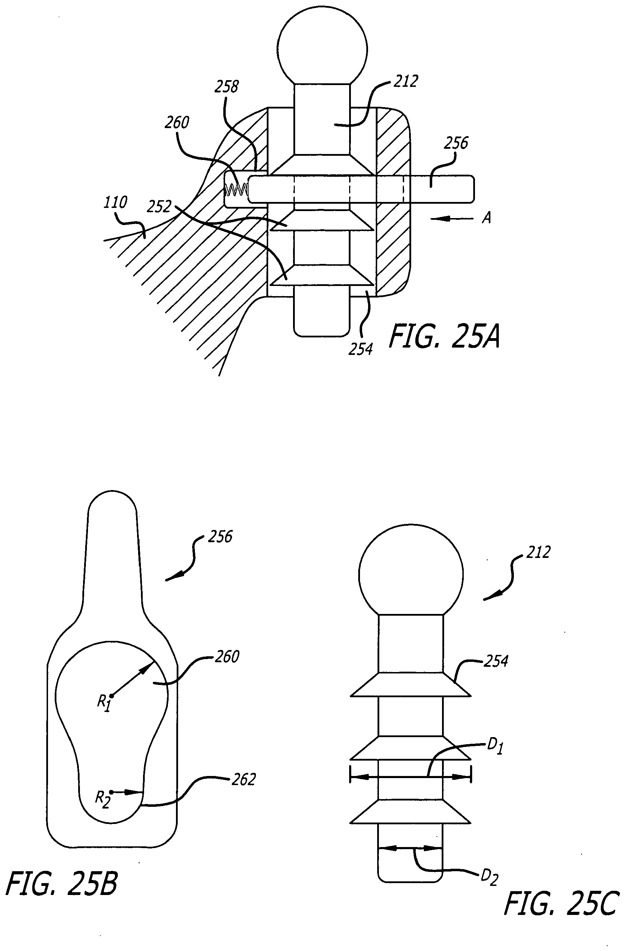

FIGS. 25A-C illustrate another approach to a mounting member 110 having a movable ball component 212. Again, in an alternate embodiment, the mounting member 110 includes a socket component in place of the movable ball component 212. In FIG. 25A, the mounting member 110 includes a ball component 212 that is movable within a main bore 254. The ball component 212 includes a plurality of rings 254 spaced apart on the shaft of the ball component. As shown in FIGS. 25A and 25C, the shaft of the ball component 212 has two diameters D₁ and D₂.

The mounting member 110 also includes a secondary bore 258 is generally perpendicular to and intersects the main bore 254. A release lever 256 is slidably mounted within the secondary bore 258. The release lever 256 is also biased in a closed (or locked) position via a spring 260 provided at end of the secondary bore 258. As shown in FIG. 25B, the release lever 256 includes two overlapping openings 260, 262 having different radii, R₁ and R₂, where R₁ > R₂.

In order to unlock the ball component 212 (i.e., allow for adjustment of the ball component), the release level is pushed in direction A, which causes the release lever to slide toward the end of the secondary bore 258. As the lever 256 slides in direction A, the smaller opening 262 is displaced in favor of the larger opening 260. As a result, the larger opening 260 is centered in the bore 254. When the larger opening 260 is centered in the bore 254, the ball component 212 is free to move within the bore 254 as the outer diameter D₁ is able to pass through the larger opening 260 of the lever 256. Once the height of the ball component 212 is adjusted, the lever 256 is released and the smaller opening 262 is centered in the bore 254 thereby locking the ball component in place.

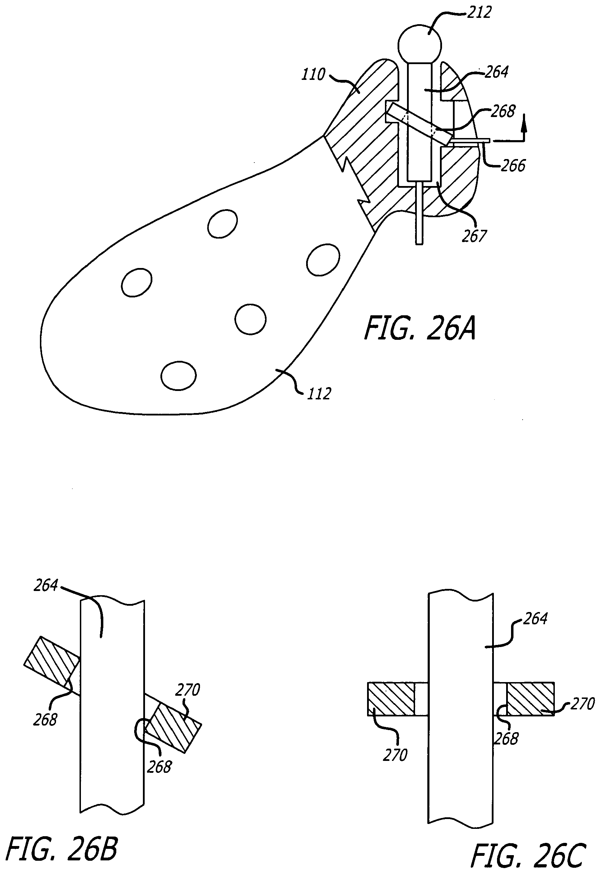

Referencing FIGS. 26A-C, a mounting member 110 having an adjustable articulating connection component includes a bore 267 that accommodates a ball component 212 having a shaft 264 coupled thereto. A socket component can replace the movable ball component 212. To adjust the height of the ball component 212, the release lever 266 is actuated upwards (direction of arrow). The upward movement of the release lever 266 articulates a washer 270 such that the inner diameter 268 of the washer is concentric with the shaft 264 as shown in FIG. 26C. Accordingly, the shaft 264 (and the ball component 212) is able to freely move upwards or downwards. On reactivation of the lever 264 (i.e., downward movement of the lever), the inner diameter 268 of the washer 270 portion of the lever is angled with respect to the shaft 264 thereby locking the shaft in place as shown in FIG. 26B.

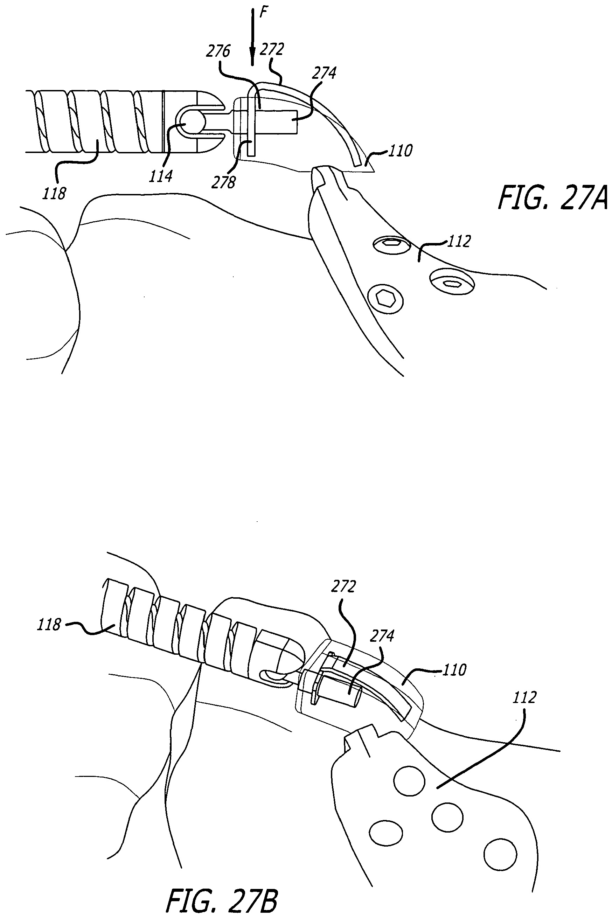

FIGS. 27A-B depict a mounting member 110 having an adjustable articulating connection similar to the mounting member shown in FIGS. 26A-C. The mounting member 110 in FIGS. 27A-B, however, includes a ball component 114 having a shaft 274 coupled thereto. In an alternate approach, the adjustable ball component 212 is substituted for a socket component. The shaft 274 includes a plurality of teeth 276 or ridges on the outer diameter. The shaft 274 can have an elliptical cross-section, but other embodiments of the shaft may have a square, circular, or other polygonal-shaped shaft.

As shown in FIGS. 27A-B, the mounting member 110 has a lever 272 that generally follows the contour of the mounting member. The large size of the lever 272 relative to the mounting member 110 allows the lever to be palpitatable through the skin after the device has been implanted, thereby allowing further adjustments after implantation. A first portion 278 of the lever 272 is generally perpendicular to the shaft 274 and includes an opening (not shown) through which the shaft passes. In a default position (i.e., locked position), the first portion of the lever 278 engages the teeth 276 on the shaft 274. When a force F is applied to the lever 272, the lever moves downward thereby making the opening of the lever concentric with the shaft 274. Accordingly, the location of the ball component 212 may be adjusted. Once the lever 272 is released, the walls of the opening on the first portion 278 of the lever engage the teeth 276 thereby locking the ball component 114 in position.

Accordingly, various embodiments of an articulating assembly which reliably and durably connects a link or an energy absorber structure to an interventional site have been described. The contemplated approaches provide durable surfaces for accomplishing articulating motion without restricting natural ranges of motion of anatomy at the interventional site. Features of certain of the disclosed approaches such as material and surface finishes as well as specific sub-structure can be incorporated into any other of the approaches to provide a patient with a desirable mount assembly and the assemblies can be employed in relevant medical as well as non-medical applications.

The various embodiments described above are provided by way of illustration only and should not be construed to limit the claimed invention. Those skilled in the art will readily recognize various modifications and changes that may be made to the claimed invention without following the example embodiments and applications illustrated and described herein, and without departing from the scope of the claimed invention, which is set forth in the following claims. The sleeve embodiments fall within the scope of the claims.

## Claims

1. An implantable, extra-articular system (10) connectable across an articulating joint, including:
a base component (12, 16, 112);
a link (20, 118); and
a mount assembly (22, 24, 110), the mount assembly including a first portion spaced from a second portion (25), the first portion configured to be locked to the base component (12, 16) and the second portion (25) configured to be attached to the link (20), wherein the link (20) is an energy absorber having load carrying capacity, **characterized in that** the system (10) further comprises a deformable sleeve (60) engageable between the mount assembly (22, 24) and the base component (12, 16) wherein the sleeve (60) is sized to be placed within a recess (50) formed in the base component (12, 16), wherein the internal bore of the sleeve (60) is configured to receive a locking stem (32) that is configured at the first portion of the mount (22, 24), such that in its assembled configuration the sleeve (60) deforms about the stem (32) and within the base recess (50).

2. The system of claim 1, wherein the sleeve (60) includes a tapered exterior.

3. The system of claim 1, wherein the sleeve (60) includes ridges formed on an exterior thereof.

4. The system of claim 1, wherein the first portion (32) comprises a tapered locking stem (32).

5. The system of claim 4, wherein the base component (12, 16, 112) includes a tapered recess (50).

6. The system according to any one of the preceding claims, wherein the mount assembly (22, 24, 110) comprises:
a body having a first end opposite a second end;
a coupling connection (116) at the first end of the mount (110, 22, 24) for coupling the mount assembly (110, 22, 24) to a base component (112, 12, 16); and
a multi-dimensional articulating connection component (114) coupled to the second end of the mount assembly (110, 22, 24),
wherein the body offsets the multi-dimensional articulating connection component (114, 25, 30) away from body anatomy when coupled to the base component (112, 12, 16) to provide an extra-articular connection, wherein the mount assembly (22, 24, 110) orients and aligns the link (20, 118) that is coupled to the multi-dimensional articulating connection component (114, 12, 16).

## Patentansprüche

1. Implantierbares extraartikuläres System (10), verbindbar über ein Knickgelenk, umfassend:
eine Basiskomponente (12, 16, 112);
ein Verbindungsstück (20, 118); und
eine Halterungsanordnung (22, 24, 110) mit einem ersten Abschnitt in einem Abstand von einem zweiten Abschnitt (25), der erste Abschnitt konfiguriert, um an der Basiskomponente (12, 16) verriegelt zu werden, und der zweite Abschnitt (25) konfiguriert, um an dem Verbindungsstück (20) befestigt zu werden, wobei das Verbindungsstück (20) ein Energieabsorber mit einer lasttragenden Kapazität ist, **dadurch gekennzeichnet, dass** das System (10) ferner eine verformbar Manschette (60) umfasst, einsetzbar zwischen der Halterungsanordnung (22, 24) und der Basiskomponente (12, 16), wobei die Manschette (60) dimensioniert ist, um innerhalb einer Vertiefung (50), gebildet in der Basiskomponente (12, 16) platziert zu werden, wobei die Innenbohrung der Manschette (60) konfiguriert ist, um einen Verriegelungsschaft (32) aufzunehmen, der am ersten Abschnitt der Halterung (22, 24) so konfiguriert ist, dass sich die Manschette (60) in der montierten Konfiguration um den Schaft (32) und innerhalb der Basisvertiefung (50) verformt.

2. System nach Anspruch 1, wobei die Manschette (60) ein kegelförmiges Äußeres hat.

3. System nach Anspruch 1, wobei die Manschette (60) Rippen umfasst, die an ihrer Außenseite gebildet sind.

4. System nach Anspruch 1, wobei der erste Abschnitt (32) ein kegelförmiges Verriegelungssystem (32) umfasst.

5. System nach Anspruch 4, wobei die Basiskomponente (12, 16, 112) eine kegelförmige Vertiefung (50) umfasst.

6. System nach einem der vorhergehenden Ansprüche, wobei die Halterungsanordnung (22, 24, 110) Folgendes umfasst:
einen Körper mit einem ersten Ende gegenüber einem zweiten Ende;
eine Kopplungsverbindung (116) an dem ersten Ende der Halterung (110, 22, 24), um die Halterungsanordnung (110, 22, 24) an eine Basiskomponente (112, 12, 16) zu koppeln; und
eine multidimensionale Knickverbindungskomponente (114), gekoppelt an das zweite Ende der Halterungsanordnung (110, 22, 24),
wobei der Körper die multidimensionale Knickverbindungskomponente (114, 25, 30), wenn sie an die Basiskomponente (112, 12, 16) gekoppelt ist, von der Körperanatomie weg verschiebt, um eine extraartikuläre Verbindung bereitzustellen, wobei die Halterungsanordnung (22, 24, 110) das Verbindungsstück (20, 118), das an die multidimensionale Knickverbindungskomponente (114, 12, 16) gekoppelt ist, orientiert und ausrichtet.

## Revendications

1. Système extra-articulaire implantable (10) pouvant être raccordé sur une articulation, comprenant :
un composant de base (12, 16, 112) ;
une liaison (20, 118) ; et
un ensemble de support (22, 24, 110), l'ensemble de support comprenant une première partie espacée d'une seconde partie (25), la première partie étant configurée pour être verrouillée sur le composant de base (12, 16) et la seconde partie (25) configurée pour être fixée à la liaison (20), dans lequel la liaison (20) est un absorbeur d'énergie ayant une capacité de support de charge, **caractérisé en ce que** le système (10) comprend en outre un manchon déformable (60) pouvant se mettre en prise entre l'ensemble de support (22, 24) et le composant de base (12, 16), dans lequel le manchon (60) est dimensionné pour être placé à l'intérieur d'un évidement (50) formé dans le composant de base (12, 16), dans lequel l'alésage interne du manchon (60) est configuré pour recevoir une tige de verrouillage (32) qui est configurée au niveau de la première partie du support (22, 24), de sorte que, dans sa configuration assemblée, le manchon (60) se déforme autour de la tige (32) et à l'intérieur de l'évidement de base (50).

2. Système selon la revendication 1, dans lequel le manchon (60) comprend un extérieur progressivement rétréci.

3. Système selon la revendication 1, dans lequel le manchon (60) comprend des crêtes formées sur son extérieur.

4. Système selon la revendication 1, dans lequel la première partie (32) comprend une tige de verrouillage progressivement rétrécie (32).

5. Système selon la revendication 4, dans lequel le composant de base (12, 16, 112) comprend un évidement progressivement rétréci (50).

6. Système selon l'une quelconque des revendications précédentes, dans lequel l'ensemble de support (22, 24, 110) comprend :
un corps ayant une première extrémité opposée à une seconde extrémité ;
un raccordement de couplage (116) au niveau de la première extrémité du support (110, 22, 24) pour coupler l'ensemble de support (110, 22, 24) au composant de base (112, 12, 16) ; et
un composant de raccordement d'articulation multidimensionnel (114) couplé à la seconde extrémité de l'ensemble de support (110, 22, 24),
dans lequel le corps décale le composant de raccordement d'articulation multidimensionnel (114, 25, 30) de l'anatomie corporelle lorsqu'il est couplé au composant de base (112, 12, 16) afin de fournir un raccordement extra-articulaire, dans lequel l'ensemble de support (22, 24, 110) oriente et aligne la liaison (20, 118) qui est couplée au composant de raccordement d'articulation multidimensionnel (114, 12, 16).
